# EUROPEAN PATENT SPECIFICATION

(11) **EP 2 061 387 B1**
(45) Date of publication and mention of the grant of the patent: **23.06.2010**
(21) Application number: 07804160.5
(22) Date of filing: 06.09.2007
(51) Int. Cl.: A61B 17/70

(54) **A BONE PLATE FOR FIXATION TO A PATIENT'S VERTEBRAE**
KNOCHENPLATTE ZUR BEFESTIGUNG AN DEN WIRBELN EINES PATIENTEN
PLAQUE VISSÉE DESTINÉE À ÊTRE FIXÉE AUX VERTÈBRES D'UN PATIENT

(30) Priority: 08.09.2006 GB 0617707
(43) Date of publication of application: 27.05.2009
(73) Proprietor: Depuy International Limited, Beeston, Leeds LS11 8DT (GB)
(72) Inventor: GOWLAND, Chris, West Yorkshire WF8 2QN (GB); UYTTENDAELE, Dirk, 9000 Gent (BE); POFFYN, Bart, 9000 Gent (BE); DE ROOSE, Joseph, 9000 Gent (BE); SANDERS, Marc, 7553 AM Hengelo (NL)
(74) Representative: Belcher, Simon James
(86) International application number: PCT/GB2007/003359
(87) International publication number: WO 2008/029142

(56) References cited:
- EP-A- 1 153 577
- FR-A- 2 704 420
- US-A- 5 674 296
- US-A1- 2002 183 757
- US-A1- 2004 006 343

## Description

This invention relates to a bone plate for fixation to a patient's vertebrae.

The ability of the spine to carry loads can be compromised by degenerative diseases, the effects of tumours, and fractures and dislocations arising from physical trauma. Elongate rigid bone plates are commonly used to stabilise the spinal column. Such bone plates are fastened to vertebrae on adjacent sides of a defect so as to span the defect. The plates are commonly fastened to the vertebrae by means of bone screws which pass through respective fixation holes in the plate. It is common for two or more screws to be used when possible, but one screw might be used when the available space is limited

When bone plates are used to treat defects in cervical vertebrae, a surgeon can use an anterior approach or a posterior approach. The choice might depend on the spinal disorder or pathology to be treated. The anterior approach in techniques in which vertebrae are fused is now popular because of the ease of approach, and the good load bearing characteristics of the plate when fastened to the spine anteriorly, providing an intrinsically stable implant, whether applied mono-cortically or bi-cortically.

An anterior approach has advantages because of the absence of anatomical structures between the incision and the spine which can significantly obstruct access. This factor means that many anterior approach plating procedures can be carried out through small incisions. Multiple small incisions might be used, for example with one incision used to delivery a plate to the spine, and another used to provide access to the plate with a driver for fixation screws.

It can be desirable to minimise the number of components that have to be manipulated independently through a small incision.

US-2002/0183757 discloses a cervical plate which is in two parts. They can be connected together in a lap joint configuration. Holes for fixing each of the parts to a respective vertebra are provided in the end of the parts.

FR2704420A discloses a plate according to the preamble of claim 1.

The present invention provides a bone plate which includes a pair of fixation pads and a bridge portion which extends between the fixation pads and is hingedly connected to at least one of the fixation pads.

Accordingly, the invention provides a bone plate as defined in claim 1.

The bone plate of the invention has the advantage that the fixation pad to which the bridge portion is hingedly connected can be manipulated when the plate is offered to a vertebra for fixation. This can remove the need for separate instrument for manipulating the fixation pad.

The bridge portion parts overlap one another in a lap joint arrangement when connected to one another. A preferred way to connect the bridge portion parts to one can involving using at least one, especially at least two, threaded fasteners. For example, one of the bridge portion parts can have at least one hole formed in it which has an internal thread. A threaded fastener can then be inserted through the other bridge portion part so that it extends into the hole with the internal thread, to engage that thread and thereby to draw the bridge portion parts together.

The bridge portion parts have interfitting formations on at least part of their facing surfaces, which allow the bridge portions to be connected to one another in one of a plurality of predetermined lengths. For example, a first one of the bridge portions can be shaped with a plurality of rounded bulbous portions spaced apart evenly along its length. The second bridge portions can define a channel with a plurality of rounded widened portions in its side wall spaced apart evenly along its length. The configuration of the bulbous portions of the first bridge portion can match the configuration of the rounded widened portions in the second bridge portion to provide a plurality of predetermined lengths of the mated bridge portions according to the number of bulbous portions which have been received in corresponding rounded widened portions. The predetermined lengths might also be provided by mating teeth on facing surfaces of the bridge portions. The interfitting formations are able to enhance the ability of the plate to withstand compressive and tensile loads.

The bridge portion will often be approximately straight along most of its length where it spans the vertebral defect. It can have a curved portion at its end where it is fastened to its fixation pad.

Preferably, at least one of the fixation pads comprises two pad portions which are hingedly connected to one another in such a way that the hinge portions are capable of relative movement so that the bone facing surface of the pad can conform to the surface of the vertebra to which the pad is to be fixed. The bone plate of the invention can therefore have the advantage that the hinged fixation pad can be offered to the surface of the vertebra to which the plate is to be fixed in such a way that the hinged pad portions can move relative to one another. This can allow the pad to conform to the surface of the vertebra. For example, when the surface of the vertebra is concave, the pad portions can adopt a configuration which might be described as a shallow "v", and when the surface of the vertebra is convex, the pad portions can adopt a configuration which might be described as a shallow "n". The bone plate of the invention can therefore facilitate fixation with good surface to surface contact between the fixation pad of the plate and the surface of the bone, especially when the plate is being manipulated through a small incision.

Furthermore, the hinged construction of the fixation pad allows the pad to collapse to facilitate implantation through a small incision.

Preferably, each of the fixation pads comprises two pad portions which are hingedly connected to one another in such a way that the hinge portions are capable of relative movement so that the bone facing surface of the pad can conform to the surface of the vertebra to which the pad is to be fixed.

The bridge portion comprises first and second bridge portion parts which are connected to respective ones of the fixation pads, and which can be connected to one another. Preferably, each of the fixation pads to which the bridge portion parts are connected comprises two pad portions which are hingedly connected to one another.

Preferably, the bone plate includes a hinge pin by which the pad portions are hingedly connected to one another and by which the bridge portion is connected to the pad.

Preferably, the surface of at least one of the fixation pads which faces towards the surface of a vertebra to which the pad is to be fixed is rounded. This can help to optimise the surface to surface contact between the fixation pad and the vertebra.

It will often be preferred for the fixation pad to be generally rectangular in outline. Its sides might be approximately straight when the pad is viewed from above. It will generally be preferred that the edge of the fixation pad is rounded at its corners. It can be preferred for the two pad portions of a fixation pad to have approximately equal size.

The fixation pad will generally have at least two holes formed in it which can receive fixation screws or other fixation elements to extend through the holes into underlying bone tissue, approximately perpendicular to the tissue. Preferably, at least one such hole is provided in each pad portion. Preferably, the angle between the axis of the hole and the bone-engaging surface of the face of the plate is at least about 60°, more preferably at least about 75° and often about 90°.

Preferably, each of the fixation pads comprises two pad portions which are hingedly connected to one another in such a way that the hinge portions are capable of relative movement so that the bone facing surface of the pad can conform to the surface of the vertebra to which the pad is to be fixed.

Components of the bone plate can be made from materials which are conventionally used in the manufacture of orthopaedic implants. Metals are generally preferred, for example titanium and certain of its alloys, and certain stainless steels.

The dimensions of the bone plate will be selected according to the intended application of the plate, including in particular the size of the vertebrae to which it is to be fastened and the distance between the vertebrae on opposite sides of the defect. Such design parameters are commonly taken into account in the design of bone plates for spinal applications.

The bone plate can be used in a surgical procedure which includes the steps of:
- fixing a first fixation plate to a first vertebra,
- fixing a second fixation plate to a second vertebra,
- providing a bridge portion which is fastened to the first and second fixation plates and which extends between the first and second vertebra.

The step of providing the bridge portion can involve connecting the first and second bridge portion parts to one another.

The method includes a step of applying a distracting or a compressing force to the vertebrae before the step of providing the bridge portion so that it extends between the first and second vertebrae. It can then be an advantage to use a bridge portion which has first and second bridge portion parts with interfitting formations on at least part of their facing surfaces, which allow the bridge portions to be connected to one another in one of a plurality of predetermined lengths. The bridge portion parts can then be connected to one another according to the spacing of the vertebrae which results from the application of the compressing or distracting force.

Embodiments of the invention will now be described by way of example with reference to the accompanying drawings, in which:
Figure 1 is an isometric view from above of the assembled bone plate according to the invention.
Figure 2 is a plan view of the bone plate shown in Figure 1.
Figure 3 is a side view sectional elevation through the bone plate shown in Figure 1.
Figure 4 is a side view of a part of the bone plate shown in Figure 1, showing collapsed hinge pads.
Figure 5 is a view along the anterior posterior axis of a spinal column on which a bone plate according to the invention has been fastened.

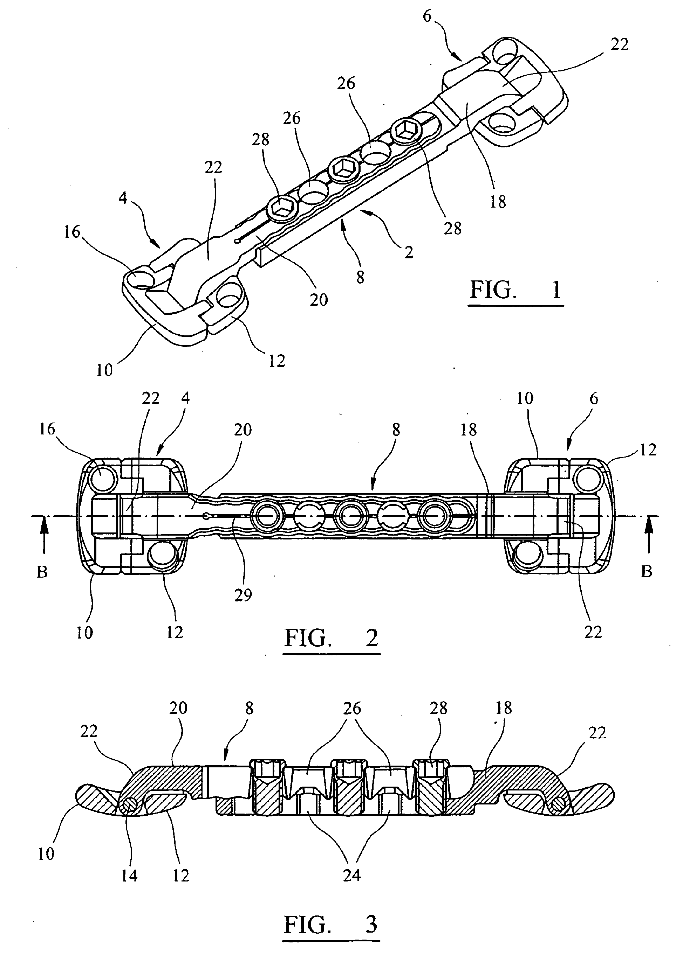

Referring to the drawings, Figures 1 to 3 show a bone plate 2 which comprises first and second fixation pads 4, 6 and a bridge portion 8 which extends between them. The first fixation pad 4 comprises first and second fixation pad portions 1,0, 12, which are hingedly connected to one another by means of a hinge pin 14. Each of the fixation pad portions 10, 12 has a fixation hole 16 in it. The fixation pad provided by the fixation pad portions is approximately square, with its corners rounded. The size of the fixation pad portions 10, 12 is approximately equal.

The bridge portion comprises first and second bridge portion parts 18, 20. Each of the bridge portion parts has a straight arm section and a curved section 22 at one end. Each of the bridge portion parts is connected at its curved end to a respective fixation pad by means of the same hinge pin 14 by which the fixation pad portions are fastened to one another.

The first bridge portion part 18 is channel shaped when viewed in cross-section along its length so that the second bridge portion part 20 can be fitted into the channel. The shape of the side walls of each of the first and second bridge portion parts 18, 20 has a wave pattern, with regular series of peaks and troughs, which enable the first bridge portion part to receive the second bridge portion part within the channel but prevent relative longitudinal movement when so received. The first bridge portion part has threaded holes 24 formed in it. The second bridge portion part has non-threaded holes 26 formed in it. Screws 28 can be inserted through the holes in the second bridge portion part to engage the threaded holes 24 in the first bridge portion part. The holes 26 can have a conical shape (when viewed in cross-section from one side) to allow the angular orientation of the screws to be adjusted.

Optionally, the second bridge part can have a slit 29 provided in it which extends between its holes 26. This can allow the bridge part to be compressed transversely provided that there are no screws in the holes. This can allow the effective length of the bone plate to be adjusted telescopically when the second bridge part is received within the first bridge part, prior to fixation of the length of the plate by placing screws in the aligned holes 24, 26 in the bridge portion parts.

The second fixation pad 6 also comprises first and second fixation pad portions 10, 12 in the same manner as the first fixation pad 4.

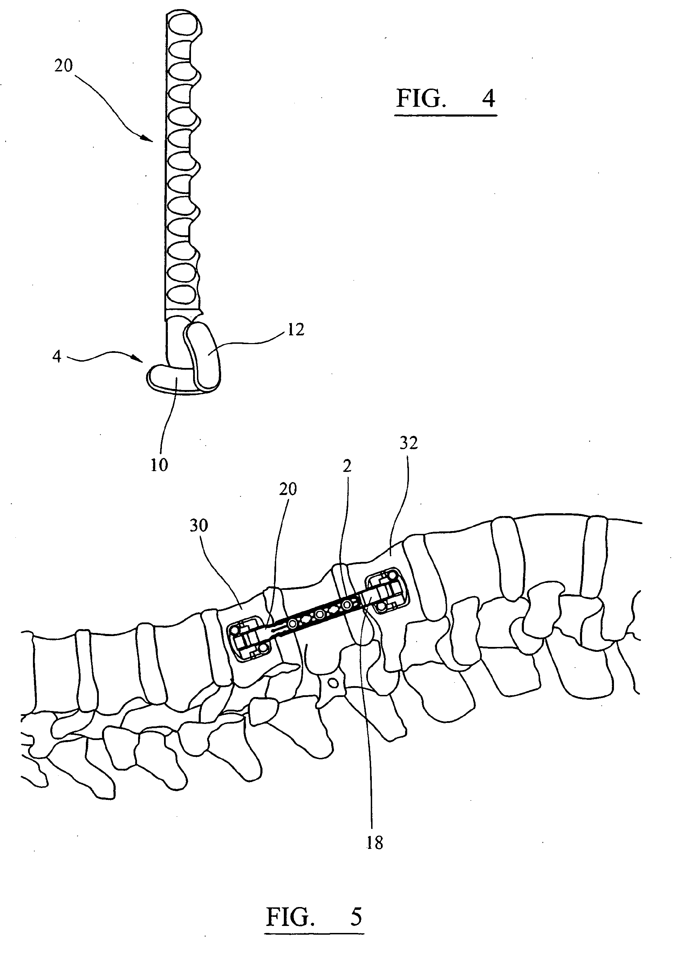

Figure 4 shows the second bridge portion part 20 from one side, where the wave pattern of the side wall is visible. The fixation pad portions 10, 12 are shown in a configuration in which they are approximately perpendicular to one another. This can facilitate insertion of the bridge portion part into a body cavity through a small incision. When the bridge portion part contacts a vertebra, the two fixation pad portions will move relative to the hinge pin 14 so that they conform to the surface of the vertebra.

Figure 5 shows a spinal column with a bone plate 2 according the invention fastened to two vertebrae 30, 32. The bone plate can be implanted using a minimally invasive anterior approach. Each of the bridge portion parts 18, 20 can be offered to the spinal column through a small incision with the fixation pad portions in a collapsed configuration as shown in Figure 4. The fixation pad portions of each fixation pad move relative to their respective hinge pins so that they conform to the bone. Each of the fixation pads can then be fastened to their respective vertebrae by means of bone screws which are passed through the fixation holes 16 in the fixation pad portions.

Distracting or compressive forces can be applied to the vertebrae 30, 32 and displacement which is imparted to the vertebrae can be retained by engagement of the wave formations of the first bridge portion part with the wave formations on the second bridge portion part and locking the bridge portion parts together by means of screws in the aligned holes 24, 26 in the bridge portion parts.

## Claims

1. A bone plate (2) for fixation to a patient's vertebrae comprising a bridge portion (8) which comprises first and second bridge portion parts (18, 20) wherein the plate includes a pair of fixation pads (4, 6) by which the plate is fixed to the vertebrae, with each of the bridge portion parts being hingedly connected to a respective one of the fixation pads so that the bridge portion extends between the fixation pads when the bridge portion parts are connected to one another **characterised in that** the first and second bridge portion parts have interfitting formations (24, 26) on at least part of their facing surfaces which allow the bridge portion parts to be connected to one another in a lap joint arrangement in one of a plurality of predetermined lengths.

2. A bone plate as claimed in claim 1, in which at least one of the fixation pads (4, 6) comprises two pad portions (10, 12) which are hingedly connected to one another in such a way that the pad portions are capable of relative movement so that the bone facing surface of the pad can conform to the surface of the vertebra to which the pad is to be fixed.

3. A bone plate as claimed in claim 1, in which each of the fixation pads (4, 6) comprises two pad portions (10, 12) which are hingedly connected to one another in such a way that the pad portions are capable of relative movement so that the bone facing surface of the pad can conform to the surface of the vertebra to which the pad is to be fixed.

4. A bone plate as claimed in claim 2 or claim 3, which includes a hinge pin (14) by which the pad portions (10, 12) are hingedly connected to one another and by which the bridge portion (8) is connected to the pad.

## Patentansprüche

1. Knochenplatte (2) für die Fixierung an den Wirbelsäulenknochen eines Patienten, mit einem Brückenbereich (8), der einen ersten und einen zweiten Brückenbereichsteil (18, 20) aufweist, wobei die Platte ein Paar Fixierauflagen (4, 6) umfasst, mittels derer die Platte an der Wirbelsäule fixiert wird, wobei jeder der Brückenbereichsteile gelenkig mit einem jeweiligen der Fixierauflagen verbunden ist, so dass sich der Brückenbereich zwischen den Fixierauflagen erstreckt, wenn die Brückenbereichsteile miteinander verbunden sind,
**dadurch gekennzeichnet, dass** der erste und der zweite Brückenbereichsteil aneinander passende Ausbildungen (24, 26) auf wenigstens einem Teil ihrer einander zugewandten Flächen haben, die es ermöglichen, dass die Brückenbereichsteile in einer überlappenden Verbindungsanordnung in einer aus einer Vielzahl vorbestimmter Länge zu verbinden sind.

2. Knochenplatte nach Anspruch 1, bei der wenigstens eine der Fixierauflagen (4, 6) zwei Auflagenbereiche (10, 12) aufweist, die gelenkig derart miteinander verbunden sind, dass die Auflagenbereiche zu einer relativen Bewegung in der Lage sind, so dass die zum Knochen weisende Fläche der Auflage sich an die Fläche des Wirbelknochens anpassen kann, an der die Auflage befestigt werden soll.

3. Knochenplatte nach Anspruch 1, bei der jede der Fixierauflagen (4, 6) zwei Auflagenbereiche (10, 12) aufweist, die gelenkig derart miteinander verbunden sind, dass die Auflagenbereiche zu einer relativen Bewegung in der Lage sind, so dass die zum Knochen weisende Fläche der Auflage sich an die Fläche des Wirbelknochens anpassen kann, an der die Auflage befestigt werden soll.

4. Knochenplatte nach Anspruch 2 oder Anspruch 3, die einen Gelenkstift (14) umfasst, mittels dem die Auflagenbereiche (10, 12) gelenkig miteinander verbunden sind und mittels dem der Brückebereich (8) mit der Auflage verbunden ist.

## Revendications

1. Plaque osseuse (2) pour la fixation sur les vertèbres d'un patient comprenant une portion de pont (8) qui comprend des première et seconde parties de portion de pont (18, 20), dans laquelle la plaque comprend une paire de patins de fixation (4, 6) grâce à laquelle la plaque est fixée sur les vertèbres, avec chacune des parties de portion de pont qui est raccordée de manière articulée à un patin respectif des patins de fixation de sorte que la portion de pont s'étend entre les patins de fixation lorsque les parties de portion de pont sont raccordées entre elles, **caractérisée en ce que** les première et seconde parties de partie de pont ont des formations de verrouillage (24, 26) sur au moins une partie de leur surface en vis-à-vis qui permettent aux parties de portion de pont d'être raccordées entre elles dans un agencement de joint par recouvrement dans une longueur d'une pluralité de longueurs prédéterminées.

2. Plaque osseuse selon la revendication 1, dans laquelle au moins l'un des patins de fixation (4, 6) comprend deux parties de patin (10, 12) qui sont raccordées de manière articulée entre elles de sorte que les parties de patin peuvent réaliser un mouvement relatif, de sorte que la surface en face de l'os du patin peut se conformer à la surface de la vertèbre sur laquelle le patin doit être fixé.

3. Plaque osseuse selon la revendication 1, dans laquelle chacun des patins de fixation (4, 6) comprend deux parties de patin (10, 12) qui sont raccordées de manière articulée entre elles de sorte que les parties de patin peuvent effectuer un mouvement relatif de sorte que la surface face à l'os du patin peut se conformer à la surface des vertèbres sur laquelle le patin doit être fixé.

4. Plaque osseuse selon la revendication 2 ou la revendication 3, qui comprend une broche d'articulation (14) grâce à laquelle les parties de patin (10, 12) sont raccordées de manière articulée entre elles et grâce à laquelle la portion de pont (8) est raccordée au patin.
